# EUROPEAN PATENT APPLICATION

(11) **EP 4 813 220 A1**
(43) Date of publication of application: **30.09.2026**
(21) Application number: 25167134.3
(22) Date of filing: 28.03.2025
(51) Int. Cl.: A23L 33/12, A23L 33/125, A23L 33/17, A23L 33/21, A23L 33/00, A61P 3/02, A61P 3/04

(54) **LIQUID MEAL REPLACEMENTS FOR WEIGHT-CONTROLLING NUTRITION**

(71) Applicant: Pretabolic Health Science GmbH, 50825 Cologne (DE)
(72) Inventor: Lück, Stephan, 95444 Bayreuth (DE); Eßfeld, Dieter, 50937 Cologne (DE); Hitz, Marco, 40211 Düsseldorf (DE)
(74) Representative: dompatent

(57) **Abstract**

The present invention provides a dietary food in liquid form, in particular a liquid meal replacement, for instance in the form of drinks, bowls, soups, sauces and pastries, for weight-controlling diets, said dietary food comprising 6 wt.% or less than 6 wt.% digestible and energy-providing carbohydrates, a protein content from 25 wt.% to 50 wt.%, both based on the weight of the total dietary food/liquid meal replacement, and at least 1 g of essential amino acid per portion of the dietary food/liquid meal replacement. The invention further provides a method for preparing such dietary food, an instant form of the dietary food, and the use of such dietary food in treating diseases connected with pathological overweight.

## Description

The present invention provides a dietary food in liquid form, in particular a liquid meal replacement, for instance in the form of drinks, bowls, soups, sauces and pastries, for weight-controlling diets, said dietary food comprising 6 wt.% or less than 6 wt.% digestible and energy-providing carbohydrates, and a protein content from 25 wt.% to 50 wt.%, both based on the weight of the total dietary food/liquid meal replacement, and at least 1 g of an essential amino acid per portion of the dietary food/liquid meal replacement. The invention further provides a method for preparing such dietary food, an instant form of the dietary food, and the use of such dietary food in treating diseases connected with pathological overweight.

### Background of the invention

A healthy lifestyle including sports and fasting is one of the core issues in modern society. In case fasting, and especially for fasting subjects with pathological overweight, a vast number of strategies and fasting regimens have been provided so far. A particular dietary food is disclosed in WO 2006081794 A1, wherein a mixture of natural fatty acids with a chain length of up to C-18 and being at least partially saturated, in the form of free fatty acids or the salts thereof or in form of monoglycerides thereof is added to food products. Such food product with added free fatty acids is used for feeding patients with food-related illnesses and illnesses caused by disturbance of the absorption, the decomposition or the elimination of certain dietary components or the secondary products thereof.

However, all dietary food products and their doses available so far are not suitable to satisfy the daily food uptake of a fasting person, i.e., are not representing a meal replacement. Relevant for this is COMMISSION REGULATION (EU) 2016/1413 of 24 August 2016, amending Regulation (EU) No 432/2012 establishing a list of permitted health claims made on foods other than those referring to the reduction of disease risk and to children's development and health. According to said meal replacement regulation a dietary food, for being a true meal replacement, must comply, among others, with a daily energy uptake between 200 and 250 kcal, a fat content of 30% or less, at least 1 g linolenic acid per meal, and from 25 to 30 % of total energy from protein. Such a meal replacement was provided in Applicant's co-pending application EP 25150899.0 (filing date 9 January 2025), in which a high content of indigestible carbohydrates, e.g. in the form of mono-, oligo- and polysaccharides, was combined with a protein content in the range of 25 to 50 wt.%. and with 6 wt.% or less than 6 wt.% digestible and energy-providing carbohydrates. The present patent application extends and complements the meal replacement formulation of EP 25150899.0 by providing a highly functional liquid or paste-like meal replacement formulation that allows for a maximally efficient uptake of the contained active substances, which is based on the finding that in certain liquid forms of the meal replacement, less indigestible carbohydrates are required.

### Summary of the Invention

Following the requirements of the Meal Replacement Regulation for weight-controlling diets and searching for a suitable meal replacement for daily single dose administration, it was further intended that the amount of digestible and energy-providing carbohydrates be less than 6 g per 100 g of the mass of the meal replacement. The aim of this reduced digestible carbohydrates is to avoid significant increases in blood glucose levels and associated insulin secretion so to allow effective fasting. It was now found that drinks or paste-like products can serve as meal replacements, which represent an innovative solution to weight-controlling nutrition concepts and connect a scientifically substantive micronutrient supply with a sensorially appealing texture and taste.

In particular, provided with the present invention is a specifically developed liquid or paste-like meal for dietetic nutrition (hereinafter shortly referred to as "composition"), especially for weight-controlling diets and metabolic optimization. The composition is based on a scientifically substantiated approach for supporting the fasting metabolism, and provides a precisely tuned combination of macronutrients, functional additives, and micronutrients, in order to ensure both an optimum nutrient intake and a sustainable saturation. The composition includes a carefully balanced equilibrium of high-quality proteins, essential fatty acids, soluble and insoluble fibers, and bioactive plant extracts and orthomolecular micronutrients. This ensures that the metabolism is actively supported, while the blood glucose level remains stable, and a sustainable energy supply is ensured. One embodiment of the composition is a Smart Balance Drink which is specifically designed to convert the body into ketogenic fasting metabolism, or stabilize the latter, in order to minimize the yo-yo effect and hunger pangs.

A further embodiment of the composition provided is a specifically developed plant-based high protein, very low carb muesli bowl, which serves as a meal replacement for a weight-controlling nutrition. The product is based on a scientifically substantive formulation, which supports metabolism during fasting and ensures an optimum nutrient supply. The composition comprises a selective macronutrient distribution with high-quality proteins, essential fatty acids, soluble and insoluble fibers, and bioactive plant extracts and orthomolecular micronutrients. The object is to ensure a sustainable saturation, to keep the blood glucose level stable, and at the same time to ensure a high bioavailability of essential nutrients.

In a first aspect, the invention thus provides a liquid or paste-like dietary food for weight-controlling diets, said liquid or paste-like dietary food comprising 6 wt.% or less than 6 wt.% digestible and energy-providing carbohydrates, having a protein content from 25 wt.% to 50 wt.%, both based on the weight of the total dietary food, and comprising at least 1 g of an essential amino acid per portion of the dietary food, in a suitable liquid or paste-like base material.

In a second aspect, the invention provides a method for preparing the dietary food of the first aspect, which comprises admixing the ingredients of the dietary food, individually or as a combined form, with the liquid or paste-like base material.

In a third aspect, the invention provides an instant form or admixture of the ingredients of the dietary food of the first aspect, i.e., void of the liquid or paste-like base material. Such instant form or admixture is suitable for use in the method of the second aspect.

In a fourth aspect, the invention provides a dietary food of the first aspect for use in treating diseases connected with pathological overweight.

In a fifth aspect, the invention provides a method for treating diseases connected with pathological overweight, said method comprising administering a subject in need of such treatment with a dietary food of the first aspect.

In a sixth aspect, the invention provides for the non-therapeutic use of the dietary food of the first aspect for fasting and reducing body weight.

### Detailed Description of the Invention

The dietary food of the first aspect of the invention comprises 6 wt.% or less than 6 wt.% of one or more digestible and energy-providing carbohydrates, preferably 5 wt.% or less than 5 wt.% of one or more digestible carbohydrates, based on the weight of the total dietary food. This corresponds to about 15 wt.% or less than 15 wt.% of one or more digestible carbohydrates of the dry weight of the dietary food, depending on the type and amount of base material used in the dietary food. The digestible and energy-providing carbohydrates according to the present invention (occasionally also designated metabolizable carbohydrates) are carbohydrates that are completely or to a certain extent broken down and metabolized by the human or animal body. The digestible carbohydrates may be in the form of mono-, oligo- and polysaccharides. Preferably digestible carbohydrates are selected from digestible carbohydrates and primarily energy-providing (mono-, di- and polysaccharides) such as vegetable starch, dextrins, various sugars (maltose, lactose, sucrose, fructose, glucose, galactose) and their sugar alcohols (such as sorbitol, maltitol, xylitol, mannitol, erythritol, maltitol, threitol and arabitol, with the exception of glycerin), in solid or dissolved form.

The dietary food of the first aspect of the invention has a protein content from 25 wt.% to 50 wt.%, preferably 25 wt.% to 45 wt.% protein, most preferably 30 wt.% to 40 wt.%, based on the dry weight of the total dietary food.

Proteins are the basic building blocks of the body and are essential for tissue formation, cell repair and enzyme production. They consist of amino acids, some of which (essential amino acids) must be obtained from food. The proteins of the invention may be obtained from the following protein sources:
Animal proteins, e.g. milk protein, whey protein, eggs, fish;
vegetable proteins, e.g. soy protein, pea protein, rice protein, hemp protein; and nuts and seeds, e.g. almonds, chia seeds, linseed.

In the present invention the following proteins are preferably used:
Milk protein, main source of high-quality protein in dietary food products, particularly rich in essential amino acids;
whey protein concentrate, provides quickly available proteins and is rich in branched-chain amino acids (BCAA);
pea protein and rapeseed protein, included in the vegan version and complement each other well to provide a complete amino acid profile;
soy protein, included in the vegan version for a higher biological value and a complete amino acid profile.

In particular, the protein in the dietary food of the first aspect of the invention is selected from animal proteins, including milk protein, whey protein, eggs and fish; vegetable proteins, including soy protein, pea protein, rice protein, hemp protein; and nuts and seeds, including almonds, chia seeds and linseed, preferably the proteins are selected from milk protein, whey protein concentrate, pea protein, rapeseed protein, and soy protein. The protein in the dietary food of the invention may also partially be derived from its liquid or paste-like base material. In line therewith, the liquid and paste-like base material suitable in the dietary food of the invention may be a milk product such as whole or skimmed milk, yoghurt and whey, and vegetable protein liquids such as soy milk.

The dietary food of the first aspect of the invention further comprises at least 1 g of an essential amino acid per portion of the dietary food. In particular, the essential fatty acid is an omega-3 and/or an omega-6 fatty acid, and preferably is selected from alpha linolenic acid, linoleic acid, and mixtures thereof.

The dietary food of the first aspect of the invention is a meal replacement. Such meal replacement may be in the form of a drink or is a paste-like dessert, bowl, soup or sauce. Furthermore or alternatively, the meal replacement may have a nutritional value from about 50 to about 100 kcal per 100 g of the dietary food. In particular, the meal replacement unit, if in the form of a drink, may have a unit volume of about 330 ml and a nutritional value of from about 200 to about 250 kcal, or, if paste like, may have a unit weight about 250 g and a nutritional value of from about 200 to about 250 kcal.

The dietary food of the first aspect of the invention may further comprise up to 15 wt.%, preferably 0.5 to 10 wt.% of non-metabolizable carbohydrates and fibers, based on the weight of the total dietary food.

Such non-metabolizable carbohydrates utilized in dietary food of the present invention (occasionally also designated non-utilizable or indigestible carbohydrates) are carbohydrates that are not or not completely broken down or metabolized by the body. They provide little or no energy and have little effect on blood sugar levels. Such non-metabolizable carbohydrates include, but are not limited to, resistant starch (RS), which is defined as "starch and starch degradation products that are not absorbed in the small intestine of healthy people". This means that RS reaches the large intestine undigested, where it is fermented by the bacteria found there (including species of the genus *Bifidobacterium* and *Lactobacillus*)*.* Thus, on the one hand, RS has physiological properties similar to those of other dietary fiber (Achour Let al., Am J Clin Nutr 66:1151-1519 (1997)).

Such non-metabolizable carbohydrates care generally occurring in the following food ingredients/components:
Fibers, e.g. chicory fiber (inulin), polydextrose, acacia fiber, oat fiber, apple fiber, citrus fiber;
sugar alcohols (polyols), e.g. maltitol, erythritol, sorbitol, isomalt, xylitol, lactitol, glycerin;
modified carbohydrates, e.g. maltodextrin, resistant dextrin, resistant maize starch; vegetable fibers, e.g. psyllium husks, guar gum, cellulose, guar fibers; and certain non-metabolizable prebiotics, e.g. fructo-oligosaccharides (FOS), galacto-oligosaccharides (GOS), isomalto-oligosaccharides (IMO).

In the present invention the following indigestible carbohydrates components are preferably used: Polydextrose, glycerin, maltitol (in chocolate ingredients), chicory fibers and maltodextrin. Such preferred non-metabolizable carbohydrates have the following properties:
Polydextrose: Polydextrose is a dietary fiber that is synthesized from glucose. It contains around 98% carbohydrates, of which almost 100% are non-metabolizable carbohydrates.
Glycerin: Glycerin is sugar alcohol that may also serve as a humectant and that is typically obtained as a by-product in the production of vegetable oils such as coconut oil. It contains about 100 % carbohydrates, of which only about 30 % are considered metabolizable carbohydrates (and are calculated in the present invention in that way), while the rest is only partially metabolized or not metabolized at all (the difference in metabolization between glycerin and maltitol is due to the fact that for glycerin the limiting factor is the enzyme activity of the liver, whereas for maltitol it is the intestinal uptake).
Maltitol: Maltitol is a sugar alcohol that is often produced by the hydrogenation of maltose from starch. It contains around 100 % carbohydrates, of which around 50 % are non-metabolizable carbohydrates, as maltitol is only partially utilized by the body. Chicory fibers: Chicory fibers (inulin) are obtained from the root of the chicory plant. They consist of about 90 % carbohydrates, of which almost 100 % are classified as non-metabolizable carbohydrates, as inulin primarily contains dietary fiber, which is hardly digested.
Maltodextrin: Maltodextrin is a modified carbohydrate produced by the partial hydrolysis of starch. It contains around 100 % carbohydrates, most of which are metabolized. Only a small proportion (~10-15%) can be considered a non-metabolizable carbohydrate, depending on branching and processing.

The dietary food of the first aspect of the invention may further comprise up to 5 wt.%, preferably from 0.3 to 3 wt% of a selective orthomolecular micronutrient supplement; and/or up to 10 wt.%, preferably 0.5 to 5 wt% functional extracts for specific health benefits, each being based on the weight of the total dietary food.

### Orthomolecular functionality in the Smart Balance Drink

This embodiment extends the disclosed dietary food so to include the integration of orthomolecular functional substances that are specifically incorporated into meal replacement products (liquid, creamy or pasty). The combination of micronutrients, bioactive plant extracts and specific macronutrients is designed to specifically support various physiological functions, including energy production, cognitive performance, muscle recovery and stress reduction.

The functionality is based on scientifically proven nutrient synergies that are integrated into the Smart Balance Drink in a standardized matrix. The composition of the orthomolecular ingredients is defined and documented for each of the following variants (the amounts per serving given in the variants refer to a serving of 330 ml). Variant 1: Smart Balance Energize - energy & performance; goal - increase in physical & mental energy, reduction of exhaustion, optimization of fat metabolism; functional ingredients (per serving): Caffeine (guarana) from about 20 to about 100 mg, preferably about 50 mg, L-carnitine from about 30 to about 200 mg, preferably about 100 mg, green tea extract (EGCG) from about 20 to about 100 mg, preferably about 50 mg, Coenzyme Q10 from about 2 to about 10 mg, preferably about 5 mg, and magnesium from about 10 to about 50 mg, preferably about 25 mg. Variant 2. Smart Balance Focus - cognitive performance & mental clarity; goal - improving concentration, mental clarity and neurological performance; functional ingredients (per serving): DHA (omega-3 fatty acid) ) from about 100 to about 500 mg, preferably about 250 mg, ginkgo biloba extract from about 20 to about 100 mg, preferably about 60 mg; phosphatidylserine from about 30 to about 200 mg, preferably about 100 mg, citicoline from about 20 to about 100 mg, preferably about 50 mg, L-tyrosine from about 30 to about 200 mg, preferably about 100 mg, caffeine (green coffee beans) from about 20 to about 100 mg, preferably about 50 mg, and vitamin B complex (B1, B6, B12) from about 10% to about 50%, preferably about 30% NRV (Nutrition Reference Values), from about 25 to about 125 µg, preferably 75 µg.

Variant 3: Smart Balance Recover - regeneration & muscle maintenance; goal - optimizing recovery after physical exertion, reducing muscle soreness; functional ingredients (per serving): BCAA-complex (leucine, isoleucine, valine, ratio about 2:1:1) from about 1 g to about 5 g, preferably about 3 g, L-glutamine from about 0.5 g to about 3 g, preferably about 1.5 g, electrolytes (sodium, potassium, magnesium) from about 30 mg to about 200 mg, preferably about 100 mg, tart cherry extract from about 30 mg to about 200 mg, preferably about 100 mg, and Vitamins C/E from about 5 to about 50 mg, preferably about 20 mg.

Variant 4: Smart Balance Calm - stress relief & relaxation; goal - reduction of stress, emotional balance & promotion of relaxation; functional ingredients (per serving): Ashwagandha extract from about 30 mg to about 200 mg, preferably about 100 mg, L-theanine from about 10 mg to about 100 mg, preferably about 50 mg, passion flower from about 30 mg to about 150 mg, preferably about 75 mg, saffron extract from about 5 mg to about 30 mg, preferably about 15 mg, and Vitamin B12 from about 0.2 µg to about 2 µg, preferably about 1 µg.

Fact sheets for the functional ingredients in Variant 1:
Caffeine (guarana): Preferred amount per serving about 50 mg; nutritional/physiological property - natural stimulant, increases alertness, reduces fatigue and can promote fat burning; synergies in the product - enhances the effect of L-carnitine in fat metabolism, combined with green tea extract for thermogenic effects; health claims "caffeine helps to increase alertness and vigilance", and "caffeine helps to improve endurance performance."

L-carnitine: Preferred amount per serving about 100 mg; nutritional-physiological property - supports the transport of fatty acids into the mitochondria, promotes fat burning and energy production; synergies in the product -supports caffeine for increased energy, boosted with coenzyme Q10 for ATP production; health claims "L-carnitine supports fat metabolism."

Green tea extract (EGCG): Preferred amount per serving about 50 mg, nutritional/physiological property - contains catechins, which have an antioxidant effect and stimulate the metabolism; synergies in the product - supports thermogenic processes with caffeine, synergizes with coenzyme Q10 for energy production; health claims "green tea polyphenols help maintain a normal metabolism."

Coenzyme Q10: preferred amount per serving about 5 mg; nutritional/physiological property - promotes mitochondrial energy production and protects against oxidative stress; synergies in the product works with magnesium to improve cell energy, boosts L-carnitine for ATP synthesis; health claims "Coenzyme Q10 contributes to normal energy metabolism."

Magnesium: Preferred amount per serving about 25 mg; nutritional/physiological property - important cofactor for over 300 enzymatic processes, muscle relaxation, energy supply; synergies in the product - supports coenzyme Q10 for cell energy, reduces cramps in combination with electrolytes; health claims "magnesium contributes to normal muscle function", "magnesium contributes to the reduction of tiredness and fatigue."

Fact sheets for the functional ingredients in Variant 2:
DHA (omega-3 fatty acid): Preferred amount per serving about 250 mg; nutritional/physiological property - important for brain function, inflammation inhibition and cardiovascular health; synergies in the product - works with phosphatidylserine for cognitive performance, combined with antioxidants for cell protection; health claims "DHA contributes to the maintenance of normal brain function."

Ginkgo Biloba Extract: Preferred amount per serving about 50 mg; nutritional/physiological property - supports blood circulation, enhances cognitive function and reduces oxidative stress; synergies in the product - combined with citicoline for improved concentration and mental performance; health claims "Ginkgo contributes to normal mental performance."

Phosphatidylserine: preferred amount per serving about 100 mg; nutritional/physiological property important membrane lipid component, supports neuronal signal transmission; synergies in the product - supports citicoline for neurotransmitter production, combined with DHA for cell integrity; health claims "phosphatidylserine contributes to the maintenance of normal cognitive function." Citicoline: Preferred amount per serving about 250 mg; nutritional/physiological property - increases acetylcholine production, supports memory and neuronal regeneration; synergies in the product - combined with ginkgo and phosphatidylserine for cognitive performance enhancement; health claims "citicoline contributes to normal cognitive function."

L-tyrosine: Preferred amount per serving about 200 mg; nutritional/physiological property - precursor of dopamine and noradrenaline, improves focus and cognitive performance; synergies in the product - enhanced with caffeine to increase alertness, supplemented with B vitamins for neurotransmitter synthesis; health claims "L-tyrosine contributes to the maintenance of normal cognitive function."

Caffeine (green coffee beans): Preferred amount per portion about 50 mg; nutritional/physiological property - contains chlorogenic acid, supports blood sugar metabolism, promotes energy production; synergies in the product - complements guarana caffeine for consistent energy release; health claims "caffeine helps improve concentration."

Vitamin B complex (B1, B6, B12): Preferred amount per serving about 30% NRV; nutritional/physiological property - essential for energy metabolism, supports the nervous system and blood formation; synergies in the product - enhanced with magnesium and coenzyme Q10 for ATP synthesis; health claims "Vitamin B6 contributes to the reduction of fatigue", "Vitamin B12 supports the normal functioning of the nervous system."

Fact sheets for the functional ingredients in Variant 3:
BCAA complex (leucine, isoleucine, valine, 2:1:1): Preferred amount per serving about 3 g; nutrition property - promotes muscle protein synthesis, reduces muscle breakdown; synergies in the product - supports L-glutamine for muscle recovery; health claims "BCAAs support the maintenance of muscle mass."

L-glutamine: Preferred amount per serving about 2 g; nutritional/physiological property - promotes muscle recovery, strengthens the immune system; synergies in the product - supplemented with BCAAs to support recovery; health claims "L-glutamine helps support the immune system".

Electrolytes (sodium, potassium, magnesium): Preferred amount per serving about 300 mg; nutritional/physiological property - regulates fluid balance, supports muscle and nerve function; synergies in the product - supports magnesium for hydration; health claims "Electrolytes help maintain a normal fluid balance".

Vitamin C/E: Preferred amount per serving Vitamin C about 30% NRV (about 24 mg) and Vitamin E about 30% NRV (about 3.6 mg); nutritional/physiological property - Vitamin C strengthens the immune system, has an antioxidant effect, supports collagen formation, and Vitamin E protects cells from oxidative stress, contributes to healthy skin; synergies in the product - in combination with B vitamins, Vitamin C improves iron absorption and Vitamin E boosts the antioxidant effect of omega-3 fatty acids; health claims "Vitamin C contributes to the normal function of the immune system" and "Vitamin E contributes to the protection of cells from oxidative stress".

Fact sheets for the functional ingredients in Variant 4:
Ashwagandha extract (Withania somnifera): Preferred amount per serving about 200 mg; nutritional/physiological property - adaptogen that reduces stress, lowers cortisol levels and promotes mental balance; synergies in the product - supports L-Theanine and passion flower for a calming effect, and together with magnesium, it promotes the reduction of stress hormones; health claims "Ashwagandha can contribute to mental relaxation" and "Ashwagandha supports resistance to stress". L-Theanine: Preferred amount per serving about 100 mg; nutritional/physiological property - promotes the formation of alpha waves in the brain, provides mental relaxation without fatigue; modulating effect on caffeine, reduces its side effects such as nervousness; synergies in the product - combined with caffeine for focused attention with less restlessness, and supplemented by passion flower for a calming effect on the nervous system; health claims "L-Theanine contributes to the maintenance of normal cognitive function".

Passion flower (Passiflora incarnata): preferred amount per serving about 50 mg; nutritional/physiological property - contains flavonoids that have a calming effect, promotes restful sleep and relaxation; synergies in the product - in combination with Ashwagandha and L-Theanine, the calming effect is enhanced, and supports electrolytes (magnesium) for a relaxing effect on the muscles; health claims "Passion flower can contribute to mental relaxation" and "Passion flower can help reduce stress".

Saffron extract (Crocus sativus): Preferred amount per portion about 30 mg; nutritional/physiological property - contains crocins and safranal, which have a mood-enhancing effect and support cognitive function; synergies in the product - boosts the effect of L-Theanine and Ashwagandha for a balanced mood, and supports vitamins B6 and B12 in their function for the nervous system; health claims "Saffron can contribute to emotional balance" and "Saffron can support cognitive function". Vitamin B12: Preferred amount per serving about 30% NRV (about 0.75 µg); nutritional/physiological property - supports the normal functioning of the nervous system and helps to reduce fatigue; essential for the formation of red blood cells; synergies in the product - together with vitamin C, supports the immune system and complements the effect of L-tyrosine and citicoline on cognitive performance; health claims "Vitamin B12 contributes to the reduction of tiredness and fatigue" and "Vitamin B12 supports the normal functioning of the nervous system".

The dietary food of the first aspect of the invention may further comprise up to 10 wt.%, preferably 0,2 to 5 wt% of a fat component. Such fat component preferably comprises (a) at least 5 wt.% polyunsaturated fatty acids and at least 2 wt.% monounsaturated fatty acids, and/or (b) at least 6 wt.% essential fatty acids; and/or (c) a linoleic acid content of at least 1 wt.%, each being based on the weight of the total dietary food. Fats according to the present invention include, but are not limited to, the following compounds:
Fats are essential nutrients that enable energy storage and supply the body with important fatty acids. They serve as a carrier for fat-soluble vitamins (A, D, E, K) and play a role in cell structure and hormone balance. Fats in food occur in the following:
Vegetable oils, e.g. sunflower oil, coconut oil, rapeseed oil, soya oil, palm oil; animal fats, e.g. butterfat, lard, fish oil;
Cocoa butter, e.g. used in chocolate products; and
nuts and seeds, e.g. almonds, walnuts, linseed, chia seeds.

In the present invention the following fats are preferably used:
Coconut oil, mainly contained in coconut bars, contains medium-chain triglycerides, which are metabolized more quickly; and
cocoa butter, contained in the chocolate ingredients (e.g. white chocolate in coconut bars and dark chocolate in other bars.

Linoleic acid: Linoleic acid is an essential omega-6 fatty acid that is important for cell membrane structure, skin health and hormone production. As the body cannot produce linoleic acid itself, it must be obtained from food.

Generic overview of linoleic acid sources:
Vegetable oils, particularly rich in sunflower oil, soya oil, corn oil and safflower oil; seeds and nuts, e.g. walnuts, linseed, chia seeds; and
certain margarine products: Often enriched with omega-6 fatty acids.

In the present invention the following linoleic acid sources are preferably used: Coconut oil, contains small amounts of linoleic acid, mainly medium-chain fatty acids, but a proportion of essential fatty acids.

The dietary food of the first aspect of the invention may further comprise fruits, dried fruits, seeds and parts thereof, preferably in an amount of 3 to 10 wt.%, based on the weight of the total dietary food.

Furthermore, the dietary food of the first aspect of the invention may comprise aroma and flavoring components, preferably in an amount of 0.1 to 2 wt.%, based on the weight of the total dietary food.

The dietary food of the first aspect of the invention may further comprise binder, preferably in an amount of 3 to 6 wt.%, based on the weight of the total dietary food. The dietary food of the first aspect of the invention may further comprise non-carbohydrate fillers, preferably in an amount of 0.5 to 10 wt.%, based on the weight of the total dietary food.

Certain additional components include substances included elsewhere, such as amino acid derivatives including proteins, and fatty acids being part of the fats.

A particular dietary food of the first aspect of the invention is a drink, notably a drink having a unit volume (volume per serving) of about 330 ml, which comprises, in a suitable liquid base, less than 6% metabolizable carbohydrates, 20% to 50% protein, essential fatty acids, including at least 1 g of linolic acid per portion, a selective orthomolecular micronutrient supplement and functional extracts for specific health benefits. It is preferred that the drink comprises 60 to 75 wt.% of a liquid whey basis, 10 to 15 wt.% whey protein isolate, 1 to 5 wt.% high omega-3 fatty acids, 1 to

5 wt.% algae oil, 1 to 10 wt.% polydextrose, 1 to 3 wt.% inulin, 1 to 3 wt.% Acacia fibers, 1 to 3 wt.% sunflower lecithin; 1 to 8 wt.% enriched fruit extract, 1 to 5 wt.% vitamin and mineral mixture and 1 to 8 wt.% orthomolecular additives, each based on the total weight of the drink.

The drink may be present in various functional variants, preferably
(i) in the variant "Energize" comprising caffeine, L-carnitine, preferably L-carnitine in an amount of about 1 g per 330 ml serving, and Q10 for energy and performance;
(ii) in the variant "Focus" comprising DHA, ginkgo biloba and phosphatidylserine for mental clarity;
(iii) in the variant "Recover" comprising BCAA, preferably a leucine/isoleucine/valine in an amount of about 3 g per 330 ml serving and a 2:1:1 mass ratio; electrolytes and vitamin C for regeneration; and
(iv) in the variant "Calm" comprising Ashwagandha, L-Theanine and saffron for stress reduction,

For more detail of said variants it is referred to the respective disclosure hereinbefore.

A further particular dietary food of the first aspect of the invention is a paste-like dietary food notably in form of a bowl having a unit weight (weight per serving) of about 250 g, which comprises, in a suitable pasty base, less than 6% metabolizable carbohydrates, 20% to 30% protein, essential fatty acids, including at least 1 g of linolic acid per portion, a selective orthomolecular micronutrient supplement and functional extracts for specific health benefits. It is preferred that the bowl comprises 60 to 70 wt.% high protein yoghurt base; 5 to 20 wt.% protein enrichment such as whey, pea or rice protein, 3 to 15 wt.% resistant starch and polydextrose, 0.05 to 1.5 wt.% sunflower lecithin, 1 to 10 wt.% functional stewed fruits, 1 to 5 wt.% fruit base such as raspberry, apricot or blackberry, 1 to 5 wt.% vitamin and mineral mixture, 0.1 to 1 wt% resistant starch & sweetener, 1 to 15 wt.% total cereal fiber including soybean crisps; lupin flakes and linseed, and 1 to 5 wt.% functional additives including orthomolecular supplements, plant extracts, adaptogens, L-carnitine, coenzyme Q10, magnesium, B vitamins, each based on the total weight of the bowl.

The bowl may be present in various functional variants, preferably
(i) the variant "Berry Boost" comprising vitamin C, zinc and elderberry extract for supports the immune system;
(ii) the variant "Apricot Power" comprising L-carnitine, magnesium and coenzyme Q10 for promoting the performance; and
(iii) the variant "Black Berry Calm" comprising Ashwagandha and L-Theanine for supporting relaxation and regeneration.

For more detail of said variants, except for the fruit components thereof, it is referred to the respective disclosure hereinbefore, the amounts given in the 330 ml servings of the drinks are to be adapted to/recalculated for the 250 g servings of the paste-like dietary product.

The second aspect of the invention refers to the production of the dietary food of the first aspect of the invention by admixing its essential components. While the preparation of a liquid food product or drink is trivial, the production of a paste-like food product may be more challenging and time-demanding and may require a particular order of steps. For example, the liquid and syrup ingredients may first be put in a batter mixer. In a second step, all the finely powdered and ready soluble ingredients are added. In a next step, digestible carbohydrates and primarily energy-providing (mono-, di- and polysaccharides) such as vegetable starch, dextrins, various sugars (maltose, lactose, sucrose, fructose, glucose, galactose) and their sugar alcohols (sorbitol, maltitol, xylitol, mannitol, erythritol, maltitol, threitol, arabitol) with the exception of glycerol, in solid or dissolved form, a syrup of indigestible (resistant starch) in combination with oligofructose are added to. These two substances are also called dietary fiber and are calculated and labeled as energy with 2 kcal/8 kJ (according to REGULATION (EU) No. 1169/2011 OF THE EUROPEAN PARLIAMENT AND OF THE COUNCIL of 25 October 2011). Due to the high fiber content in the bar, this meets the prescribed energy content for a meal replacement for weight control nutrition.

As a further ingredient, almond paste is added, which is also the fat-supplying source. The fat of the almond is characterized by a very high content of monounsaturated and polyunsaturated fatty acids. In addition, the puree also serves as a natural source of fiber. As a natural ingredient, almonds also possess essential minerals and trace elements. The prescribed content of 1 g of linoleic acid is more than met by the almond paste. The amount of almond paste was determined so that the regulation regarding the maximum fat content is also met here. Next, pea protein is added. Since this example is a vegan bar, the protein comes from peas. Due to the low biological value of pea protein, the amino acid N-acetyl-L-cysteine is added in a defined amount to comply with the prescription for a meal replacement for weight control nutrition.

Last but not least, other ingredients such as minerals and vitamins, coarse material and fruit components are added in the prescribed quantity, aroma-imparting substances and, to protect the contained fat from oxidation, the antioxidant tocopherol acetate is added in the necessary quantity and the entire mass is kneaded well. To supplement and optimize the nutritional content and technological property, algae oil (improving the fatty acid pattern) is added.

The dietary food, if utilized in the third or fourth aspect of the invention may further contain additional pharmaceutically active ingredients and/or pharmaceutically acceptable carrier and filler.

The invention is described at the hand of the following examples, which are not to be construed as limiting the invention.

### Examples

### Preparation of a Drink or Paste-like Food (Bowl)

For preparing the drink or bowl the respective ingredients of the dietary food are individually or in a combined form admixed, in a suitable mixing device, with the liquid or paste-like base material. In case one or more of the ingredients are insoluble or are available in a coarse form, the soluble ingredients and fine ingredients are admixed with the base material in one or more initial mixing steps, followed by additional steps with the coarse or insoluble ingredients. The mixing time for each step is generally in the rage of 1 to 10 min at room temperature, or below room temperature (5 to 20 °C). The resulting drink or bowl is then filled in appropriate container for consumption and/or storage. As a final step, the drink or bowl may be cooled or frozen for storage.

### Determination of Nutritional Value of the Food Product

The nutritional values of the resulting Food product are calculated based on the individual product specifications and/or the general information from the Federal Food Code.

### Example 1: Smart Balance Drink

A typical product for the invention is a balance drink based on the general recipe shown in Table 1 which is prepared according to the general procedure set forth hereinbefore:

**Table 1: Composition of the Smart Balance Drink**

| Ingredient | Amount (%) | Function |
|---|---|---|
| Whey (ultrahigh temperature treated, low fat) | 68.50% | Liquid base |
| Whey protein isolate | 12.00% | High quality protein source |
| High omega-3 fatty acids (algae oil with DHA/EPA) | 2.00% | Source of essential fatty acids |
| Polydextrose (fiber & texture-providing agent) | 3.00% | reduces metabolizable carbohydrates, improves texture & creaminess |
| Inulin (prebiotic fibers) | 1.00% | optimizes texture, provides creaminess |
| Acacia fibers (soluble fibers) | 1.00% | optimizes texture, provides creaminess |
| Emulsifier (sunflower lecithin) | 1.50% | stabilizes the oil-in-water emulsion, improves the absorption of lipid-soluble vitamins, provides linolic acid |
| Enriched fruit extract | 3.00% | contains antioxidative polyphenols, supports cell protection and regeneration, natural flavor source |
| Vitamin and mineral mixture | 3.00% | provides 30% of the daily requirement of essential micronutrients according to NRV, reduces deficiency symptoms |
| Functional ingredients (orthomolecular additives) | 5.00% | contains bioactive plant extracts and micronutrients for selective metabolic support |

**Table 2: Nutritional values per 100 g and per 330 ml**

| Nutrient | per 100 g | per 330 ml | Percentage per total calory mass |
|---|---|---|---|
| Energy (kJ/kcal) | 295 kJ/70 kcal | 973 kJ/231 kcal | 100% |
| Fat (g) | 1.90 g | 6.27 g | 25% |
| of which saturated fatty acids (g) | 0.34 g | 1.12 g | 5% |
| Carbohydrates (g) | 3.00 g | 9.90 g | 17% |
| of which sugars (g) | 0.98 g | 3.23 g | 6% |
| of which polyhydric alcohols (g) | 0.00 g | 0.00 g | 0% |
| Fibers (g) | 12.10 g | 39.93 g | 12% |
| Protein (g) | 25.10 g | 82.83 g | 40% |
| Salt (g) | 0.10 g | 0.33 g | 0.50% |
| Linolic acid (g) | 0.40 g | 1.32 g | 3% |

### Example 2: Smart Balance Breakfast

The balance breakfast with the recipe shown in Table 3 below was prepared according to the general procedure set forth above.

**Table 3: Composition of the Smart Balance Breakfast**

| Ingredient | Amount (%) | Function |
|---|---|---|
| High protein yoghurt base | 65.40% | Liquid base, provides creaminess & pleasant consistency |
| Protein enrichment (whey, pea, rice protein - added in yoghurt) | 9.80% | High quality proteins for saturation, muscle development & conservation of muscle mass |
| Resistant starch and polydextrose (fibers & texture-providing agents) | 5.30% | Reduces metabolizable carbohydrates, improves texture & provides creamy consistency |
| Emulsifier (sunflower lecithin) | 0.30% | Stabilizes the oil-in-water emulsion, improves the absorption of lipid-soluble vitamins |
| Functional stewed fruits (total) | 6.30% | provides natural fruit flavors, antioxidants & supports cell protection |
| Fruit base (raspberry, apricot or blackberry) | 3.40% | Base for flavor & natural sweetness |
| enriched with 30% of the daily dose of vitamins and minerals | 2.40% | provides essential micronutrients according to NRV |
| added resistant starch & sweeteners | 0.50% | provides desired consistency & mild sweetness |
| Very low carb muesli total | 9.95% | Source of high-quality proteins and fibers, provides crispiness & texture |
| Soybean crisps | 5.37% | Plant protein & crispy texture |
| Lupin flakes | 3.41% | Low fat protein source for mouth feeling & structure |
| Linseed (kibbled, for linolic acid fraction) | 1.17% | Source of linolic acid, fibers & structure |
| Functional additives (orthomolecular supplements, plant extracts, adaptogens, L-carnitine, coenzyme Q10, magnesium, B vitamins) | 2.95% | selectively supports metabolism & vital functions |

**Table 4: Nutritional values per 100 g and per 250 g portion**

| Nutrient | per 100 g | per 250 g (portion) | Percentage per total calory mass |
|---|---|---|---|
| Energy (kJ/kcal) | 360 kJ/86 kcal | 900 kJ/216 kcal | 100% |
| Fat (g) | 2.60 g | 6.50 g | 27.10% |
| of which saturated fatty acids (g) | 0.48 g | 1.20 g | 5.00% |
| Carbohydrates (g) | 4.85 g | 12.13 g | 22.50% |
| of which sugars (g) | 3.50 g | 8.75 g | 16.20% |
| Fibers (g) | 12.00 g | 30.00 g | 11.10% |
| Protein (g) | 18.50 g | 46.25 g | 39.80% |
| Salt (g) | 0.10 g | 0.25 g | - |
| Linolic acid (g) | 0.40 g | 1.00 g | - |

## Claims

1. A liquid or paste-like dietary food for weight-controlling diets, said liquid or paste-like dietary food comprising 6 wt.% or less than 6 wt.% digestible and energy-providing carbohydrates, having a protein content from 25 wt.% to 50 wt.%, both based on the weight of the total dietary food, and comprising at least 1 g of an essential amino acid per portion of the dietary food, in a suitable liquid or paste-like base material.

2. The dietary food of claim 1 or 2, which further comprises
(i) up to 15 wt.%, preferably up to 10 wt.% of non-metabolizable carbohydrates and fibers; and/or
(ii) up to 5 wt.%, preferably from 0.3 to 3 wt% of a selective orthomolecular micronutrient supplement, and/or
(iii) up to 10 wt.%, preferably from 0.5 to 5 wt% functional extracts for specific health benefits, each being based on the weight of the total dietary food.

3. The dietary food of claim 1 or 2, which is a meal replacement, preferably
(i) is in the form of a drink or is a paste-like dessert, bowl, soup or sauce; and/or
(ii) has a nutritional value from about 50 to about 100 kcal per 100 g of the dietary food; and/or
(iii) a meal replacement unit, if in the form of a drink, has a unit volume of about 330 ml and a nutritional value of from about 200 to about 250 kcal; and/or
(iv) a meal replacement unit, if paste like, has a unit weight about 250 g and a nutritional value of from about 200 to about 250 kcal; and/or
(v) the liquid or paste like base material is a milk product such as whole or skimmed milk, yoghurt and whey, and vegetable protein liquids such as soy milk.

4. The dietary food of any one claims 1 to 3, wherein
(i) the dietary food comprises 5 wt.% or less than 5 wt.% of digestible carbohydrates, based on the weight of the total dietary food; and/or
(ii) the digestible carbohydrates are in the form of mono-, oligo- and polysaccharides, preferably are selected from digestible carbohydrates and primarily energy-providing (mono-, di- and polysaccharides) such as vegetable starch, dextrins, various sugars (maltose, lactose, sucrose, fructose, glucose, galactose) and their sugar alcohols (sorbitol, maltitol, xylitol, mannitol, erythritol, maltitol, threitol, arabitol) with the exception of glycerol, in solid or dissolved form.

5. The dietary food of any one of claims 1 to 4, wherein
(i) the dietary food comprises 25 wt.% to 45 wt.% protein, preferably 30 wt.% to 40 wt.% protein, based on the weight of the total dietary food; and/or
(ii) the protein is selected from animal proteins, including milk protein, whey protein, eggs and fish; vegetable proteins, including soy protein, pea protein, rice protein, hemp protein; and nuts and seeds, including almonds, chia seeds and linseed, preferably the proteins are selected from milk protein, whey protein concentrate, pea protein, rapeseed protein, and soy protein; and/or
(ii) wherein the essential amino acid is selected from alpha linolenic acid, linoleic acid, and mixtures thereof.

6. The dietary food of any one of claims 1 to 5, wherein
(i) the non-metabolizable carbohydrates are selected from fibers, including chicory fiber, polydextrose, acacia fiber, oat fiber, apple fiber, citrus fiber; sugar alcohols, including maltitol, erythritol, sorbitol, isomalt, xylitol, lactitol, glycerin; modified carbohydrates, including maltodextrin, resistant dextrin, resistant maize starch; vegetable fibers, including psyllium husks, guar gum, cellulose and guar fibers; and non-metabolizable prebiotics, including fructo-oligosaccharides, galacto-oligosaccharides and isomalto-oligosaccharides, preferably the non-metabolizable carbohydrates are selected from polydextrose, glycerin, maltitol, chicory fibers and maltodextrin; and/or
(ii) the selective orthomolecular micronutrient supplement comprises (a) amino acids and amino acid derivatives, and/or (b) minerals and vitamins, and/or
(iii) the functional extracts for specific health benefits are selected from green tea extract, ginkgo biloba extract, tart cherry extract, Ashwagandha extract and saffron extract.

7. The dietary food of any one of claims 1 to 6, further comprising
(i) up to 10 wt.% of a fat component, said fat component preferably (a) comprising at least 5 wt.% polyunsaturated fatty acids and at least 2 wt.% monounsaturated fatty acids, and/or (b) comprising at least 6 wt.% essential fatty acids; and/or (c) having a linoleic acid content of at least 1 wt.%; and/or
(ii) fruits, dried fruits, seeds and parts thereof, preferably in an amount of 3 to 10 wt.%;
(iii) aroma and flavoring components, preferably in an amount of 0.1 to 2 wt.%;
(iv) humectants and/or binder, preferably in an amount of 3 to 6 wt.%,
(v) non-carbohydrate fillers, preferably in an amount of 0.5 to 10 wt.%,
each being based on the weight of the total dietary food.

8. The dietary food of any one of claims 1 to 7, which is a drink comprising, in a suitable liquid base, less than 6% metabolizable carbohydrates, 20% to 50% protein, essential fatty acids, including at least 1 g of linolic acid per portion, a selective orthomolecular micronutrient supplement and functional extracts for specific health benefits, preferably the drink comprises 60 to 75 wt.% of a liquid whey basis, 10 to 15 wt.% whey protein isolate, 1 to 5 wt.% high omega-3 fatty acids, 1 to 5 wt.% algae oil, 1 to 10 wt.% polydextrose, 1 to 3 wt.% inulin, 1 to 3 wt.% Acacia fibers, 1 to 3 wt.% sunflower lecithin; 1 to 8 wt.% enriched fruit extract, 1 to 5 wt.% vitamin and mineral mixture and 1 to 8 wt.% orthomolecular additives, each based on the total weight of the drink.

9. The dietary food of claim 8, wherein the drink is present in various functional variants, preferably
(i) the variant "Energize" comprising caffeine, L-carnitine, preferably L-carnitine in an amount of about 1 g per 330 ml serving, and Q10;
(ii) the variant "Focus" comprising DHA, ginkgo biloba and phosphatidylserine;
(iii) the variant "Recover" comprising BCAA, preferably a leucine/isoleucine/valine in an amount of about 3 g per 330 ml serving and a 2:1:1 mass ratio; electrolytes and vitamin C; and
(iv) the variant "Calm" comprising Ashwagandha, L-Theanine and saffron.

10. The dietary food of any one of claims 1 to 7, which is a paste-like dietary food in form of a bowl, comprising, in a suitable pasty base, less than 6% metabolizable carbohydrates, 20% to 30% protein, essential fatty acids, including at least 1 g of linolic acid per portion, a selective orthomolecular micronutrient supplement and functional extracts for specific health benefits, preferably the bowl comprises 60 to 70 wt.% high protein yoghurt base; 5 to 20 wt.% protein enrichment such as whey, pea or rice protein, 3 to 15 wt.% resistant starch and polydextrose, 0.05 to 1.5 wt.% sunflower lecithin, 1 to 10 wt.% functional stewed fruits, 1 to 5 wt.% fruit base such as raspberry, apricot or blackberry, 1 to 5 wt.% vitamin and mineral mixture, 0.1 to 1 wt% resistant starch & sweetener, 1 to 15 wt.% total cereal fiber including soybean crisps; lupin flakes and linseed, and 1 to 5 wt.% functional additives including orthomolecular supplements, plant extracts, adaptogens, L-carnitine, coenzyme Q10, magnesium, B vitamins.

11. The dietary food of claim 10, wherein the bowl is present in various functional variants, preferably
(i) the variant "Berry Boost" comprising vitamin C, zinc and elderberry extract;
(ii) the variant "Apricot Power" comprising L-carnitine, magnesium and coenzyme Q10; and
(iii) the variant "Black Berry Calm" comprising Ashwagandha and L-Theanine.

12. A method for preparing the dietary food of any one of claims 1 to 11 which comprises admixing the ingredients of the dietary food, individually or as a combined form, with and into the liquid or paste-like base material.

13. An instant form or admixture of the ingredients of the dietary food of any one of claims 1 to 11.

14. A dietary food of any one of claims 1 to 11 for use in treating diseases connected with pathological overweight.

15. A non-therapeutic use of the dietary food of any one of claims 1 to 11 for fasting and reducing body weight.
